(19) Europäisches Patentamt / European Patent Office / Office européen des brevets

(11) **EP 4 670 709 A1**

(12) **EUROPEAN PATENT APPLICATION**

(43) Date of publication:
**31.12.2025 Bulletin 2026/01**

(21) Application number: **25184629.1**

(22) Date of filing: **23.06.2025**

(51) International Patent Classification (IPC):
**A61K 9/00** *(2006.01)*  **A61K 9/06** *(2006.01)*
**A61K 47/36** *(2006.01)*  **A61P 31/00** *(2006.01)*

(52) Cooperative Patent Classification (CPC):
**A61K 9/0034; A61K 9/06; A61K 47/36; A61P 31/00**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH LA MA MD TN**

(30) Priority: **24.06.2024 IT 202400014464**

(71) Applicant: **Nirial Pharma S.r.l.**
**20123 Milano (IT)**

(72) Inventors:
• **MUNDI, Gaetana Giulia**
  **Milano (IT)**
• **DEL GIUDICE, Andrea**
  **20123 Milano (IT)**

(74) Representative: **Braidotti, Andrea et al**
**Praxi Intellectual Property S.p.A.**
**Via F. Baracca, 5/A**
**30173 Venezia (IT)**

(54) **MUCOADHESIVE COMPOSITION FOR VAGINAL USE**

(57) The present invention describes a composition for vaginal, preferably intravaginal, use for the prevention and treatment of vaginal conditions, with mucoadhesive, soothing, moisturizing and pH-regulating properties. By remaining adherent to the vaginal mucosa for prolonged periods, the composition according to the invention is therefore able to alleviate pain, burning and itching, often resulting from vaginal dryness, to promote the rebalancing of the vaginal bacterial flora, to restore the natural pH, to combat vaginal atrophy, vaginosis, vaginitis, to reduce local irritation of the tissues and to combat the proliferation of pathogens.

VG = Vaginal Gel SO3101 Batch: RS52123, **SLS** = Sodium lauryl sulfate 0.5%, **NC** = Negative Control

**Figure 3**

EP 4 670 709 A1

**Description**

Technical field

The present invention describes a mucoadhesive composition for vaginal use to restore the natural pH, soothe pain, burning and itching, promote the rebalancing of the vaginal flora and combat vaginal dryness.

Known art

The vagina is one of the most delicate and sensitive surfaces identifiable on the female body, and for this reason it is vulnerable to a wide variety of conditions that cause discomfort and malaise in the affected individual. Although the vagina is in fact an internal membrane of the organism, the numerous physiological activities wherein it is directly involved subject it to constant and inevitable exposure to the surrounding environment, transforming it into an attractive target for biological and non-biological contaminants. The proximity to the orifices for the completion of physiological needs, the direct involvement in the excretion of menstrual blood, the rubbing with external bodies during sexual intercourse, sometimes prolonged contact with internal tampons or contraceptive devices make the vagina susceptible to attack by pathogenic aggressors capable of jeopardizing the health of the vagina itself. Careful and conscious personal hygiene is not always sufficient to prevent the appearance of irritations and/or infections that can alter/compromise the well-being and life quality of the affected person.

Among those responsible for the health and functional balance of the vagina is the vaginal flora, i.e. a microbiome consisting essentially of some strains of lactobacilli that colonize the most superficial layer of the vaginal epithelium, protecting it from pathogenic aggressors from the surrounding environment. These beneficial bacteria, through their metabolic activity, first hydrolyze and then transform glycogen into glucose and lactic acid. The local production of the latter is essential in maintaining the natural pH of the vagina acidic so as to inhibit the proliferation of pathogenic microorganisms. Not only that, but the population of lactobacilli itself antagonizes the colonies of undesired bacterial guests, preventing their growth. Since an imbalance in the vaginal flora leads to a drastic lowering of the body's immune defenses, preventive care of the beneficial bacterial colony appears necessary to prevent the onset of genital infections. Alterations in the population of vaginal bacteria can be caused, for example, by hormonal imbalances or disorders, by a lack of intimate hygiene as well as by its abuse (especially if aggressive detergents are used), or by physical and/or psychological stress. Sometimes it is also excessively tight and synthetic clothing that locally raises the temperature, creating an ideal microenvironment for the development of microbes.

Once the balance of the bacterial flora is compromised, resulting from qualitative or quantitative alterations of the microorganisms involved, the vaginal mucosa, which remains unprotected, can easily be affected by infections of various kinds, for example fungal (e.g. candida), bacterial (e.g. staphylococcal infection) or viral (e.g. genital herpes).

Another situation of deep discomfort can arise following lesions, irritations or abrasions of the vaginal mucosa due to excessive dryness or following intercourses consumed in the absence of adequate lubrication. The small lacerations that open on the vaginal membrane can not only cause deep pain and burning but in fact represent access points to the circulatory system for germs and microorganisms.

Finally, another common condition of discomfort that female gender individuals frequently report is vaginal dryness. The onset of this situation of malaise is often caused by a condition of atrophy, that is, a reduction in the thickness of the mucosa following hormonal disorders (e.g. estrogen deficiency). The lack of adequate and natural epithelial lubrication, and the alteration of the composition of the hydrolipidic film that covers the vaginal membrane cause numbness, itching, phlogosis and sometimes slight bleeding in the affected subject.

Some formulations relating to the treatment of vaginal disorders have been reported in literature, in particular:

CN 108 452 293 A describes a composition comprising hyaluronic acid (2 g/2.02% w/w), lactic acid (3 g/3.03% w/w), levulinic acid (3 g/3.03% w/w), luteolin (5 g/5.05% w/w), basic fibroblast growth factor (1 g/1.01% w/w), glycogen (5 g/5.05% w/w), thickener (8 g/8.08% w/w) mixture of carbomer (carbopol) and hydroxyethylcellulose, Ethyl p-hydroxybenzoate (5 g/5.05% w/w) and purified water (67 g/67.67% w/w) capable of regulating and maintaining the normal acid environment of the vagina, inhibiting bacterial growth, lubricating, moisturizing and combating vaginitis; and

CN 108 158 995 A reports a composition in the form of a foam comprising 2 parts of quaternary ammonium chitosan (1.62%), 5 parts of chitosan (4.06%), 2 parts of sodium hyaluronic acid (1.62%), 0.1 part of 1,3-butanediol (0.081%), 1 part of oat beta-glucan (0.81%), 2 parts of natural lactic acid (1.62%), 5 parts of glycerin (4.06%), 1 part of foaming agent, 105 parts of purified water (85.29%), capable of acting as an antibacterial against vagino-genic pathogens and for the treatment of vaginitis, cervicitis and other gynecological inflammations.

Unless expressly excluded, this chapter is to be construed as part of the detailed description according to the present invention.

Technical problem

Products known for vaginal use for the prevention and/or treatment of diseases affecting the vagina are commonly constrained and limited to a limited efficacy over time due to the inability to remain adherent to the mucosa. The moist and self-cleaning epithelial surface of the vagina causes a natural excretion of the applied formulations, a characteristic that significantly reduces the contact time and therefore the soothing and/or moisturizing efficacy of the product itself.

Furthermore, known products offer rather limited solutions to the many situations of discomfort experienced by the individual, requiring in most cases the combined use of multiple compositions to tampon and comprehensively resolve all the problems that arise in the vaginal canal. In fact, numerous products created for the treatment of symptoms associated with bacterial or fungal infections allow the control of the proliferation of pathogens but at the same time induce or exacerbate collateral problems such as, for example, mucosal dryness and dehydration. It therefore appears evident, especially in terms of *compliance,* the need to develop a composition that can simultaneously combat conditions of vaginal dryness, intimate discomfort and pain, itching and local inflammation through its properties of soothing, moisturizing, rebalancing of the vaginal flora and restoration of the natural pH with the aim of inducing an overall state of well-being of the vaginal mucosa, resolving multiple situations of *discomfort* in a complete and comprehensive manner.

Summary

The object of the present invention is a composition for vaginal use for the prevention and/or treatment of vaginal conditions.

This mucoadhesive composition for vaginal use comprises:

- at least one mucoadhesive agent in a quantity between 0.01% and 15% w/w or v/v, preferably between 0.1% and 5% w/w or v/v,
- at least one moisturizing agent in a quantity between 0.01% and 15% w/w or v/v preferably between 0.1% and 5% w/w or v/v,
- at least one pH regulator in a quantity between 0.01% and 10% w/w or v/v, preferably between 0.01% and 5% w/w or v/v,
- at least one agent for the control of bacterial proliferation in a quantity between 0.001% and 8% w/w or v/v, preferably between 0.001% and 5% w/w or v/v,
- at least one agent for the rebalancing of the vaginal bacterial flora in a quantity between 0.001% and 8% w/w or v/v, preferably between 0.001% and 5% w/w or v/v,
- at least one diluent or solvent in a quantity sufficient to reach 100%, wherein,

    said composition has a pH between 3-5,
    said at least one agent for the control of bacterial proliferation is chosen from:

    • ferulic acid in a quantity between 0.01-5% w/w or v/v;
    • resveratrol in a quantity between 0.01-5% w/w or v/v; and
    • combinations thereof;

    and said agent for the rebalancing of the bacterial flora comprises or consists of by sodium carboxymethyl betaglucan in a quantity between 0.001-5% w/w or v/v.

It is an object of the present invention also a composition comprising:

- sodium hyaluronate with a molecular weight preferably chosen between 100-5000 kDa and respective mixtures, in a quantity between 0.1-5% w/w;
- glycogen in a quantity between 0.01-8% w/w;
- lactic acid in a quantity between 0.01-5% w/w;
- sodium carboxymethyl betaglucan in a quantity between 0.001-5% w/w;
- at least one agent for the control of bacterial proliferation;
- a diluent and/or solvent in a quantity sufficient to reach 100% w/w; wherein,
    said composition has a pH between 3-5 and said at least one agent for the control of bacterial proliferation is chosen from:

- ferulic acid in a quantity between 0.01-5% w/w,
- resveratrol in a quantity between 0.01-5% w/w; and
- combinations thereof.

The composition according to the invention has mucoadhesive properties, such properties allow the composition itself to remain adherent to the vaginal mucosa while performing soothing, emollient, moisturizing, natural pH restoration and bacterial flora rebalancing activities.

Various embodiments of the composition are therefore an object of the invention.

The present invention also relates to the methods of administration of the composition which is particularly indicated to combat situations of vaginal discomfort caused by atrophy, dryness, irritation, vaginitis, vaginosis, dysbiosis, pain and vaginal itching.

Furthermore, an object of the invention is a kit comprising the composition, a suitable applicator and instructions for use.

These and further objects will be evident from the detailed description below.

Description of the figures

Fig. 1: Instrumental evaluation with Franz Cell.
Fig. 2: 3D vaginal epithelium model grown in vitro.
Fig. 3: Histogram for assessing irritation potential.
Fig. 4: Cell viability as a function of the administered dose.

Definitions

- According to the present invention, TEER, measured in $\Omega$-cm$^2$, is defined as the electrical resistance of a multicellular layer that allows to monitor the quality of the cultured epithelium and to evaluate its optimal barrier function.
- According to the present invention, an "active" ingredient is defined as a pharmaceutically and cosmetically acceptable ingredient capable of expressing a biological functionality.
- In this document, the percentages (%) referring to the quantities of the individual components constituting the composition of the invention are to be understood as % by mass (% mass on mass, % w/w) or as % by volume (% volume on volume, % v/v), unless otherwise specified.
- In this document, the terms "about" or "around" as used herein when referring to a measurable value such as a quantity, a duration of time, and the like, are intended to encompass variations of $\pm 20\%$, $\pm 10\%$, $\pm 5\%$, $\pm 1\%$, or $\pm 0.1\%$ from the specified value, where such variations are appropriate for performing the methods described.

Description

The object of the present invention is a composition for vaginal use for the prevention and/or treatment of vaginal conditions.

Preferably, the composition of the invention is suitable for intravaginal use. The composition according to the invention promotes the local release of its active ingredients and allows them, thanks to their mucoadhesive properties, to perform their mechanical activity for a prolonged period of time so as to combat the multiple situations of vaginal, preferably intravaginal, discomfort and/or alteration.

By means of the expression of mucoadhesive properties, in fact, the composition according to the present invention is capable of remaining superficially adhered to the vaginal membrane, allowing a gradual and controlled release of the active ingredients contained therein. In fact, despite the secretion of body fluids and the physiological hormonal lubrication that keep the vagina clean but at the same time moist, the composition according to the invention is advantageously capable of exhibiting a tenacious and prolonged adherence to the mucosa of the vaginal canal, opposing viscous resistance to natural excretion.

Advantageously, the composition according to the invention is capable of combating the sensation of vaginal dryness by remoisturizing and protecting the mucosa through its humectant properties.

Furthermore, the composition according to the invention expresses soothing properties against local pain, relieves burning, attenuates intimate itching, reduces turgor and counteracts tissue irritation.

In addition, the composition according to the invention advantageously allows to combat both infections affecting the vagina such as vaginitis and vaginosis and conditions of alterations of the vaginal mucosa, caused for example by vaginal atrophy.

In particular, the composition according to the present invention is advantageously capable of restoring the natural pH of the vagina and of promoting the rebalancing of the vaginal bacterial flora.

The composition of the invention allows the vagina itself to regain the environmental conditions suitable for a state of good health.

The inventors of this invention have identified in the combination of carboxymethyl betaglucan and glycogen a pair of ingredients capable, in doses appropriately studied as described below, of promoting the rebalancing of the vaginal bacterial flora. These ingredients, supported by the pH of the composition, allow the vaginal mucosa to re-establish the ideal environmental conditions for a state of good health.

In particular, the composition according to the present invention is capable of promoting the restoration of lactobacilli colonies by supporting their growth and the consequent repopulation of the vaginal membrane.

The pair of ingredients carboxymethyl betaglucan and glycogen is also able to promote/induce the gelation of the composition of the invention. The chemical-physical properties conferred by the gelled formulation of the composition of the invention allow said composition to perform more effectively the soothing, mucoadhesive, moisturizing, protective and vaginal bacterial flora rebalancing activities.

The inventors have in fact verified/demonstrated that the mucoadhesive properties of the composition are primarily conferred by the presence of the combination of glycogen and carboxymethyl betaglucan. Compared to the compositions of the known art, these two ingredients are able to confer to the composition of the invention a greater adhesive capacity and a longer duration over time on the moist mucosae of the vagina.

The composition of the invention is further capable of performing an antibacterial activity. The combination of appropriately selected ingredients and then added according to certain quantitative ranges allows to combat the proliferation of pathogenic microorganisms.

Furthermore, the composition according to the invention applied on the affected area generates a thin mucoadhesive and protective film of the underlying epidermis, while performing soothing and emollient activities.

The composition according to the present invention comprising:

- at least one mucoadhesive agent,
- at least one moisturizing agent,
- at least one pH regulator,
- at least one agent for the control of bacterial proliferation,
- at least one agent for rebalancing the vaginal bacterial flora,
- at least one diluent and/or solvent

wherein the at least one mucoadhesive agent may be selected by way of non-limiting example and from the group comprising hyaluronic acid (both low and high molecular weight and mixtures thereof), xanthan gum, polyacrylic acid (e.g., CARBOPOL®), hydroxyethylcellulose, wherein each mucoadhesive agent has a molecular weight between 100 - 5000 KDalton.

The at least one moisturizing agent may be selected by way of non-limiting example from the group comprising hyaluronic acid (both low and high molecular weight and mixtures thereof), xanthan gum, polyacrylic acid (e.g., CARBOPOL®), hydroxyethylcellulose, where each moisturizing agent has a molecular weight between 100 - 5000 Kdalton,

the at least one pH regulator can be chosen by way of non-limiting example from the group comprising lactic acid, malic acid, citric acid, acetic acid.

The at least one pH regulator is chosen in order to obtain a composition with a pH between 3-5.

The at least one agent for controlling bacterial proliferation can be chosen by way of non-limiting example from the group comprising resveratrol, ferulic acid, betaglucan, preferably carboxymethylbetaglucan, lactic acid.

In one embodiment, the at least one agent for the control of bacterial proliferation is resveratrol.

In another embodiment, the at least one growth-control agent is ferulic acid.

In a further embodiment, the at least one proliferation control agent is a combination of resveratrol and ferulic acid.

In a way not deducible from the known art, the inventors have surprisingly observed how resveratrol and/or ferulic acid, in combination with beta-glucan, preferably sodium carboxymethyl beta-glucan, are effective in promoting antiproliferative activity against bacterial pathogens. Beta-glucan, enhanced by the presence of glycogen, gives the composition a gel-like consistency that improves adhesion to the mucosal surface and ensures a controlled release of resveratrol and/or ferulic acid, allowing them to best exert their antibacterial function.

The inventors further observed that resveratrol is also able to support the carboxymethylbetaglucan and glycogen couple in rebalancing the vaginal bacterial flora.

The at least one agent for the rebalancing of the vaginal bacterial flora can be chosen by way of non-limiting example from the group comprising betaglucan, preferably carboxymethylbetaglucan, glycogen, resveratrol.

The at least one diluent and/or solvent is any pharmaceutically or cosmetically acceptable diluent and/or solvent and is chosen for example by way of non-limiting example from the group comprising water, silicone, vegetable oils.

The composition according to the present invention comprises:

- at least one mucoadhesive agent in a quantity between 0.01-15% w/w or v/v, preferably between 0.1-5% w/w or v/v;
- at least one moisturizing agent in a quantity between 0.01-15% w/w or v/v, preferably between 0.1-5% w/w or v/v;
- at least one pH regulator in a quantity between 0.01-10% w/w or v/v, preferably between 0.01-5% w/w or v/v;
- at least one agent for the control of bacterial proliferation in a quantity between 0.001-8% w/w or v/v, preferably between 0.001-5% w/w or v/v;
- at least one agent for the rebalancing of the vaginal bacterial flora in a quantity between 0.001-8% w/w or v/v, preferably between 0.001-5% w/w or v/v;
- at least one diluent or solvent in a quantity sufficient to reach 100% w/w or v/v.

In one embodiment, the composition of the invention comprises:

- at least one mucoadhesive agent in a quantity between 0.01% and 15% w/w or v/v, preferably between 0.1% and 5% w/w or v/v,
- at least one moisturizing agent in a quantity between 0.01 % and 15% w/w or v/v preferably between 0.1% and 5% w/w or v/v,
- at least one pH regulator in a quantity between 0.01% and 10% w/w or v/v, preferably between 0.01% and 5% w/w or v/v,
- at least one agent for the control of bacterial proliferation in a quantity between 0.001% and 8% w/w or v/v, preferably between 0.001% and 5% w/w or v/v,
- at least one agent for the rebalancing of the vaginal bacterial flora in a quantity between 0.001% and 8% w/w or v/v, preferably between 0.001% and 5% w/w or v/v,
- at least one diluent or solvent in a quantity sufficient to reach 100%, wherein,

  said at least one agent for the control of bacterial proliferation is chosen from:

  - ferulic acid in a quantity between 0.01-5% w/w or v/v;
  - resveratrol in a quantity between 0.01-5% w/w or v/v; and
  - combinations thereof;

  and said agent for the rebalancing of the bacterial flora comprises or is constituted by sodium carboxymethyl betaglucan in a quantity between 0.001-5% w/w or v/v.

In one embodiment, the composition of the invention comprises:

- sodium hyaluronate with a molecular weight preferably selected between 100-5000 kDa and mixtures thereof, in a quantity between 0.1-5% w/w or v/v;
- glycogen in a quantity between 0.01-8% w/w or v/v;
- lactic acid in a quantity between 0.01-5% w/w or v/v,
- sodium carboxymethyl betaglucan in a quantity between 0.001-5% w/w or v/v;
- a diluent and/or solvent in a quantity sufficient to reach 100% w/w or v/v; and
- at least one of ferulic acid in a quantity between 0.01-5% w/w or v/v, resveratrol in a quantity between 0.01-5% w/w or v/v; and combinations thereof;

wherein,
said composition has a pH between 3-5.

The composition according to the present invention may further comprise at least one antioxidant agent.

The at least one antioxidant agent may be chosen by way of non-limiting example from the group comprising tocopherol, tocopherol acetate, lycopene, ascorbic acid, resveratrol, ferulic acid, coenzyme Q10, whether in neutral form, salt form, in complex form or in any other chemically, pharmaceutically and cosmetically acceptable form.

The composition according to the present invention further comprising the at least one antioxidant agent in a quantity between 0.01-5% w/w or v/v.

The composition according to the present invention may further comprise at least one pharmaceutically and cosmetically acceptable additive. Such additive may be, by way of non-limiting example, selected from the group of pharmaceutically and/or cosmetically acceptable preservatives, pH regulators, texturizers and excipients.

The at least one additive may be chosen by way of non-limiting example from the group comprising propanediol, polysorbate 20, 1,2-hexanediol, caprylyl glycol, tropolone, disodium phosphate, polysorbate 60, sodium phosphate.

The composition according to the present invention further comprising the at least one additive in a quantity between 0.0001% - 5 % w/w or v/v.

In one embodiment, the composition of the invention comprises:

- sodium hyaluronate with a molecular weight preferably chosen between 100-5000 kDa and mixtures thereof, in a quantity between 0.1-5% w/w or v/v;
- glycogen in a quantity between 0.01-8% w/w or v/v;
- lactic acid in a quantity between 0.01-5% w/w or v/v,
- sodium carboxymethyl betaglucan in a quantity between 0.001-5% w/w or v/v;
- at least one agent for the control of bacterial proliferation;
- a diluent and/or solvent in a quantity sufficient to reach 100% w/w or v/v;
  wherein,
  said composition has a pH between 3-5 and said at least one agent for the control of bacterial proliferation is chosen from:

  • ferulic acid in a quantity between 0.01-5% w/w or v/v,
  • resveratrol in a quantity between 0.01-5% w/w or v/v; and
  • combinations thereof.

In a preferred embodiment, the composition according to the invention comprises:

- sodium hyaluronate (with a molecular weight preferably chosen between 100-5000 kDa, even more preferably between 200-1000 kDa and relative mixtures) in a quantity between 0.1-5% w/w or v/v;
- glycogen in a quantity between 0.01-8% w/w or v/v;
- ferulic acid in a quantity between 0.01-5% w/w or v/v,
- lactic acid in a quantity between 0.01-5% w/w or v/v,
- resveratrol in a quantity between 0.01-5% w/w or v/v,
- sodium carboxymethyl betaglucan in a quantity between 0.001-5% w/w or v/v,
- a diluent and/or solvent in a quantity sufficient to reach 100% w/w or v/v.

The sodium hyaluronate according to the invention may consist of a mixture of low molecular weight sodium hyaluronate and high molecular weight sodium hyaluronate, selected from molecular weights between 100-5000 KDa.

In a still more preferred embodiment, said composition further comprises at least one antioxidant agent.

The at least one antioxidant agent may be chosen by way of non-limiting example from the group comprising tocopherol, tocopherol acetate, lycopene, ascorbic acid, resveratrol, ferulic acid, coenzyme Q10, whether in neutral form, salt form, in complex form or in any other chemically, pharmaceutically and cosmetically acceptable form.

The composition according to the present invention further comprising the at least one antioxidant agent in a quantity between 0.01-5% w/w or v/v.

The above written composition may further comprise at least one pharmaceutically and cosmetically acceptable additive. Such additive may be chosen by way of non-limiting example from the group of pharmaceutically and/or cosmetically acceptable preservatives, pH regulators, texturizers and excipients.

The at least one additive may be chosen by way of non-limiting example from the group comprising propanediol, polysorbate 20, 1,2-hexanediol, caprylyl glycol, tropolone, disodium phosphate, polysorbate 60, sodium phosphate.

The composition according to the present invention further comprising the at least one additive in a quantity between 0.0001- 5 % w/w or v/v.

The following compositions are particularly preferred.

**Composition n1**

- sodium hyaluronate, with a molecular weight between 200-500 kDa and mixtures thereof, is present in a quantity between 0.1-2.00% w/w or v/v, preferably approximately 0.50% w/w or v/v;
- glycogen is present in a quantity between 0.04-0.80% w/w or v/v, preferably about 0.20% w/w or v/v;

- Ferulic acid is present in a quantity between 0.05-0.30% w/w or v/v, preferably about 0.15% w/w or v/v;
- lactic acid is present in a quantity between 0.02-0.20% w/w or v/v, preferably about 0.09% w/w or v/v;
- resveratrol is present in a quantity between 0.05-0.30% w/w or v/v; preferably about 0.08% w/w or v/v;
- sodium carboxymethyl betaglucan is present in a quantity between 0.001-0.01 % w/w or v/v, preferably about 0.0021% w/w or v/v;
- propanediol is present in a quantity between 1.00-5.00% w/w or v/v;
- polysorbate-20 is present in a quantity between 0.5-3.00% w/w or v/v;
- hydroxyethyl cellulose is present in a quantity between 0.1-2.00% w/w or v/v;
- 1,2-hexanediol is present in a quantity between 0.01-1.50% w/w or v/v;
- caprylyl glycol is present in a quantity between 0.01-1.50%w/w or v/v;
- tropolone is present in a quantity between 0.0002-0.02% w/w or v/v;
- disodium phosphate is present in a quantity between 0.0005-0.02% w/w or v/v;
- polysorbate-60 is present in a quantity between 0.0005-0.02% w/w or v/v;
- sodium phosphate is present in a quantity between 0.0001-0.01% w/w or v/v;
- water is present in a quantity necessary to reach 100% w/w or v/v.

**Composition n2**

- sodium hyaluronate, with a molecular weight between 200-500 kDa and related mixtures, is present in a quantity between 0.1-2.00% w/w or v/v, preferably approximately 0.50% w/w or v/v;
- glycogen is present in a quantity between 0.04-0.80% w/w or v/v preferably about 0.20% w/w or v/v;
- lactic acid is present in a quantity between 0.02-0.20% w/w or v/v, preferably about 0.09% w/w or v/v;
- resveratrol is present in a quantity between 0.05-0.30% w/w or v/v, preferably about 0.08% w/w or v/v;
- sodium carboxymethyl betaglucan is present in a quantity between 0.001-0.01% w/w or v/v, preferably about 0.0021% w/w or v/v;
- propanediol is present in a quantity between 1.00-5.00% w/w or v/v,
- polysorbate-20 is present in a quantity between 0.5-3.00% w/w or v/v,
- hydroxyethyl cellulose is present in a quantity between 0.1-2.00% w/w or v/v;
- 1,2-hexanediol is present in a quantity between 0.01-1.50% w/w or v/v;
- caprylyl glycol is present in a quantity between 0.01-1.50% w/w or v/v;
- tropolone is present in a quantity between 0.0002-0.02% w/w or v/v;
- disodium phosphate is present in a quantity between 0.0005-0.02% w/w or v/v;
- polysorbate-60 is present in quantities between 0.0005-0.02% w/w or v/v;
- sodium phosphate is present in a quantity between 0.0001-0.01% w/w or v/v;
- water is present in a quantity necessary to reach 100% w/w or v/v.

**Composition n3**

- sodium hyaluronate, with a molecular weight between 200-500 kDa and related mixtures, is present in a quantity between 0.1-2.00% w/w or v/v, preferably approximately 0.50% w/w or v/v;
- glycogen is present in a quantity between 0.04-0.80% w/w or v/v, preferably about 0.20% w/w or v/v;
- ferulic acid is present in a quantity between 0.05-0.30% w/w or v/v, preferably about 0.15% w/w or v/v;
- lactic acid is present in a quantity between 0.02-0.20% w/w or v/v, preferably about 0.09% w/w or v/v;
- sodium carboxymethyl betaglucan is present in a quantity between 0.001-0.01% w/w or v/v, preferably about 0.0021% w/w or v/v;
- propanediol is present in a quantity between 1.00-5.00% w/w or v/v;
- polysorbate-20 is present in a quantity between 0.5-3.00% w/w or v/v;
- hydroxyethyl cellulose is present in a quantity between 0.1-2.00% w/w or v/v;
- 1,2-hexanediol is present in a quantity between 0.01-1.50% w/w or v/v;
- caprylyl glycol is present in a quantity between 0.01-1.50% w/w or v/v;
- tropolone is present in a quantity between 0.0002-0.02% w/w or v/v;
- disodium phosphate is present in a quantity between 0.0005-0.02% w/w or v/v;
- polysorbate-60 is present in a quantity between 0.0005-0.02% w/w or v/v;
- sodium phosphate is present in a quantity between 0.0001-0.01% w/w or v/v;

- water is present in a quantity necessary to reach 100% w/w or v/v.

Conveniently, the sodium hyaluronate in each of the compositions may consist of a mixture of low molecular weight sodium hyaluronate and high molecular weight sodium hyaluronate, selected from molecular weights ranging from 100-5000 KDa.

The composition according to the present invention is in the form of a gel.

The present invention also comprises formulations comprising the composition of the invention in the form of lotion, cream, ointment, paste, salve, ovules.

The composition according to the present invention has a pH between 3-5.

The composition according to the present invention has a viscosity (measured by Rotavisc Viscometer ME-VI S000, Large Clean Room, IKA, 2023, 101047942) between 10,000 and 90,000 cPs. The present viscosity range allows the composition of the invention to express an efficient and long-lasting mucoadhesiveness.

The composition according to the present invention can be formulated in the form of gel, lotion, cream, ointment, paste, salve, ovules.

The composition according to the present invention is formulated for vaginal use, *i.e.* to be applied, spread and distributed homogeneously on the surface of the vaginal mucosa.

Preferably, the composition of the invention is for intravaginal use.

Advantageously, the composition according to the invention exhibits a percentage of mucoadhesiveness (as described below according to the "method for evaluating mucoadhesive efficacy") higher than 80%, preferably higher than 85%, preferably higher than 90%, preferably higher than 95%, more preferably 97%.

Advantageously, the composition according to the invention has a film-forming effect (or barrier effect) once applied to the mucosa, preferably vaginal mucosa, more preferably intravaginal mucosa.

Advantageously, the composition according to the invention does not exhibit sensitizing properties.

Advantageously, the composition according to the invention does not have irritating properties for the epithelium of the vaginal mucosa.

Advantageously, the composition according to the invention possesses preservative properties that inhibit/slow down the growth of pathogenic microorganisms over time.

The composition according to the present invention for vaginal use allows to combat conditions of vaginal dryness, intimate discomfort and pain, itching and local inflammation through its soothing, moisturizing, vaginal flora rebalancing and natural pH restoring properties.

The composition according to the present invention for vaginal use, preferably intravaginal, is capable of performing all the above-mentioned properties in an exhaustive and efficient manner by means of a proven mucoadhesive capacity.

The inventors have demonstrated how the chemical-physical properties, conferred by the combination of selected ingredients and the state of the matter (for example, the gel), allow the composition of the invention to express a markedly increased mucoadhesiveness. Compared, for example, to a liquid or in a form of foam composition, the gel of the composition of the present invention guarantees a high-performance adhesiveness to the vaginal mucosa for prolonged times. Not only that, but a foam would be difficult to apply intravaginally, while a liquid would tend to detach from the mucosa too quickly. On the contrary, a gel formulation appears to be the ideal compromise to achieve the desired objectives.

The inventors have observed that the composition of the invention shows superior efficacy both antibacterial and in the regeneration of vaginal flora. These surprising performances are attributable to the combination of the gelled physical state of the composition-obtained through a rigorous qualitative and quantitative selection of the ingredients-and the presence of resveratrol and/or ferulic acid. In fact, the latter, alone or in combination, when combined with beta-glucan and glycogen, are more efficient in expressing their antiproliferative properties on bacteria and in promoting the restoration of vaginal flora.

Various characteristics of the composition were evaluated: the mucoadhesive capacity, i.e. the ability of the composition to remain adhered to the mucosa for a time sufficient to carry out its function, and the film-forming capacity, i.e. the ability to create a film, a barrier capable of protecting the mucosa itself.

The mucoadhesive capacity was evaluated on reconstructed tissues of human vaginal mucosa by evaluating the percentage of inhibition of the lectin-glycoprotein bond. Said method uses an indirect technique to identify substances that are capable of binding to the surface of vaginal mucosa cells by inhibiting the lectin-glycoprotein bond.

The evaluation of the mucoadhesive capacity of the composition of the present invention according to the described method comprises the use of a colorimetric assay and will be described in detail in the examples.

The test result showed that the composition according to the invention is highly mucoadhesive on the vaginal mucosa, reaching 97% of composition adhered to the mucosa after 10 hours from application. This persistence on the mucosa allows the moisturizing and film-forming agents to perform their function on the mucosa itself.

In addition to the evaluation of the mucoadhesive capacity, the film-forming capacity of the composition was also evaluated. The film-forming properties of the composition according to the invention allow to obtain a high degree of protection of the mucosa from damages that may be caused by external agents or pathogens.

The evaluation of the film-forming capacity of the composition of the present invention according to the described method comprises the use of a specific test based on the use of trypan blue and is described in detail in the examples. The tests carried out have highlighted a high film-forming activity that remains significant up to at least 10 hours from the start of the test, thus allowing to obtain with a single application an excellent protection time of the mucosa.

The inventors, in order to evaluate the safety of the composition according to the invention, have carried out a biocompatibility study for the in vitro evaluation of the cytotoxicity of the composition according to the invention in accordance with the ISO 10993-12:2021 and UNI EN ISO 10993-5:2009 standards.

The cytotoxic effect of the composition according to the invention on cell cultures is measured by means of a cell viability test with Neutral Red Uptake (NRU). The NRU test measures cell viability, based on the cell's ability to incorporate the Neutral red dye.

If the relative cell viability of the sample (pure or 100% extracted) is $\geq$70% of the control group, the material can be considered non-cytotoxic. A reduction in cell viability of more than 30% indicates the potential cytotoxicity of the composition. The values collected from in vitro cytotoxicity tests remain an indication of the in vitro potential toxicity. The % of cell viability recorded on the cell sample under examination after treatment with the vaginal gel according to the invention was found to be 38.42%, thus attributing the vaginal composition potential cytotoxicity. In fact, lactic acid can affect the osmotic balance of cells. An accumulation of lactic acid can alter the normal balance of ions and molecules within the cell, causing osmotic stress. This stress can cause cell contraction, cell dysfunction and, ultimately, cell death, therefore in vitro cytotoxicity does not translate into negative effects on the vaginal mucosa in clinical use.

The irritation potential and the sensitization capacity of the composition according to the invention were also evaluated, in order to assess its application potential for the purposes described above.

The test that allowed to evaluate the irritation potential was carried out using cultures of human vaginal epithelial cells reconstituted in vitro to form a three-dimensional tissue. The Uni EN ISO 10993-5 standard and the $ET_{50}$ parameter (treatment time with the substance corresponding to 50% epithelial cell mortality) were used as a reference. The test allowed to quantitatively establish the effects of the tested product on cell proliferation through the cell viability test by staining with MTT. This colorimetric method allowed to obtain an indirect measurement of cell viability through the ability of a mitochondrial enzyme, succinate dehydrogenase, to metabolize a substrate such as tetrazolium salts.

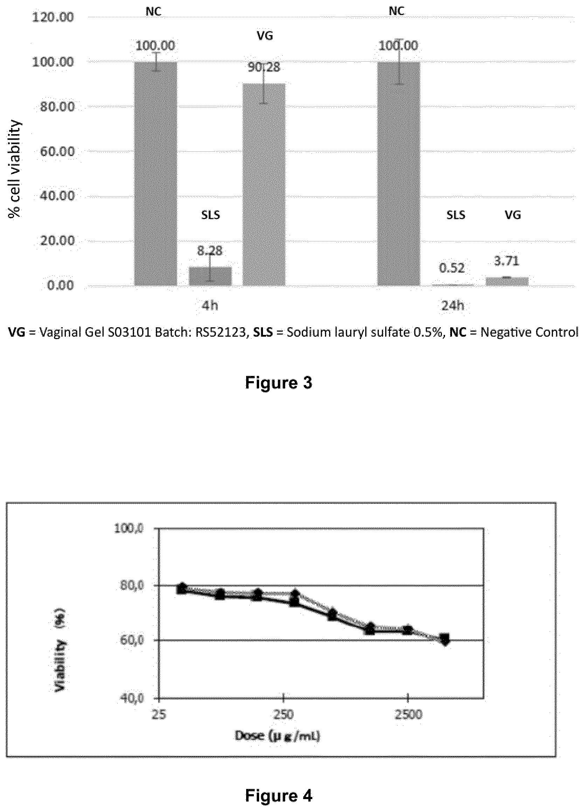

The tests, carried out as detailed in the examples, provided good results (Table 13, 14 and Figure 3), taking into account the methods of use and the persistence of the device as is in the vaginal area, also evaluating the behaviour of a slightly irritating substance such as SLS at 0.5%, it can be stated that the composition according to the invention has not shown irritating effects towards the human vaginal epithelium under normal conditions of use.

The test to evaluate the sensitization potential is instead carried out by investigating the activation capacity of h-CLAT (human Cell Line Activation test), the test aims to discriminate whether substances or mixtures are skin sensitizers or not, in accordance with the UN GHS classification. The test was conducted on a monocyte cell line called THP-1, since this cell type is strongly involved in the immune responses of the skin, the elective target organ of topical products. On these cells, the modulation of the expression of two co-stimulatory molecules, CD54 and CD86, is evaluated using 2,4-dinitrochlorobenzene (DNCB), a typical contact sensitizer, as a positive control.

The increase in the expression of these co-stimulatory molecules on the monocyte is therefore a sign of activation of an immune response following exposure to a potentially allergenic antigen. Functionally, in fact, the expression of co-stimulatory molecules on this cell type corresponds to the acquisition of competence for the presentation of the antigen in the elective site (the skin in our case) wherein in vivo contact sensitization clinically manifests itself.

The results of the study of cell viability as a function of dose are reported in Table 1 and Figure 4 and show that, at the concentrations of cutaneous use of the composition, cell viability remains high and the expression of the signal substances CD86 and CD54, measured by determination of RFI%, remains low. (Table 17)

As can be seen from the data reported, the vaginal gel according to the present invention does not show any sensitizing potential.

The efficiency of the preservative system of the composition according to the invention was then evaluated using the Challenge test, a predictive method suitable for evaluating the efficacy of a preservative system used in the formulation of a non-sterile cosmetic or pharmaceutical product.

By reproducing in the laboratory the conditions of microorganism pollution to which products may be subjected during processing, storage and normal use by the consumer, valid indications on the stability of the product are obtained. Since the preparation of the product does not take place in sterile conditions, there is always a level of basic microbial contamination that must be kept under control by an adequate preservative system. The use of the product by the consumer also brings further contamination that is repeated over time throughout the period of use of the product. During the test performed in the laboratory, there is a tendency to deliberately exaggerate the experimental conditions by inoculating the product with a very high concentration of microorganisms, which is difficult to verify in reality. The product was inoculated with different strains of microorganisms, as reported in the examples, and the percentage of reduction of the microbial load was evaluated at different times. The strains used to study the preservation potential of the composition according to the present invention were *Pseudomonas aeruginosa* (ATCC 9027), *Staphylococcus aureus* (ATCC 6538), *Candida albicans* (ATCC 10231), Aspergillus brasiliensis (ATCC 16404).

This test is conducted in accordance with what is described in the European Pharmacopoeia edition 9.0 in paragraph 5.1.3. (Efficacy of antimicrobial preservation).

The test allows to evaluate the dynamics of the reduction of the microbial load starting from the moment of inoculation. The effectiveness of the preservative system was measured in particular at 2, 7, 14 and 28 days after inoculation. The composition according to the present invention satisfies the most stringent requirements for the evaluation of the efficiency of the preservative system. In particular, a reduction of post-inoculation microbial growth of at least 2 logs after 2 days, of at least 3 logs after 7 days for bacteria without any subsequent regrowth on day 28 and of at least 2 logs, for fungi, after 14 days without any subsequent regrowth on day 28 is considered suitable for a preparation for topical use, according to the most restrictive A criteria.

The composition according to the invention, suitably stored in an intact, hermetic and product-compatible container (e.g., 22x135 aluminium tube) after 270 days both stored at room temperature (25°) and stored in conditions of higher ambient temperature (40°C), has shown excellent preservation properties.

Indeed, the proliferation of pathogens as *Candida Albicans, Staphylococci aureus, Pseudomonas aeruginosa* was not detected.

The chemical-physical characteristics such as appearance (clear or slightly opalescent gel), colour (pale yellow), odour (undefined) and pH (approx. 4.0) underwent slight alterations, resulting in them being non-significant. Finally, the efficiency of the preservative system was evaluated during the use of the finished product. In particular, a test was performed to determine the degree of bacterial reduction during a simulation of use of the vaginal gel (applied via a cannula) and by introducing microbial contamination with some bacterial and fungal strains (Escherichia coli, Staphylococcus aureus and Candida albican) for a period of 30 days. A more detailed description is given in the "Examples" section. From the tests conducted, the vaginal gel according to the invention was found to have an effective reduction of all the tested strains during the entire simulation test (30 days of use with the applicator) with the cannula washing procedure described in the "Examples" section.

The inventors have therefore created a composition with high mucoadhesive properties, capable of remaining adherent to the vaginal epithelium for at least ten hours and of forming a film to protect the mucosa itself; the composition also comprises active ingredients with emollient and moisturizing action and active ingredients capable of maintaining the correct pH of the vaginal environment.

The composition according to the invention has also shown a low sensitizing power, does not have irritating or allergenic properties if used on mucous membranes, and has excellent preservative power. The composition according to the invention therefore has the ability to combat conditions of vaginal dryness, intimate discomfort and pain, itching and local inflammation through its soothing, moisturizing, rebalancing of the vaginal flora and restoration of the natural pH properties.

The composition according to the present invention has proven effective in the treatment and improvement of the symptoms of Vaginitis and Vaginosis, which are the most common infections by microorganisms of the vaginal mucosa. They are characterized by an increase in pH and an alteration of the normal vaginal flora that can lead to vaginal disorders such as itching, burning, swelling and vaginal discharge, in some cases with an unpleasant odor.

The composition according to the present invention has also proven effective in the treatment and improvement of the symptoms of Vaginal Atrophy or atrophic vaginitis, a condition characterized by lack of elasticity and turgor of the skin, dryness and inflammation of the vaginal walls. Atrophy does not only affect the thickness of the epithelium, but makes the mucosa more susceptible to lesions, increases the local pH and alters the resident bacterial flora, thus increasing the risk of infections.

It is a further object of the present invention a kit comprising the composition according to the invention, in predefined dosage units, a reusable or disposable applicator containing effective doses of the composition and instructions for use.

**EXAMPLES**

The examples given below are intended as exemplary embodiments and are not intended to limit the scope of the present invention.

All examples were made with the compositions described above and in particular with the preferred composition n1

**Determination of mucoadhesion.**

The procedure for evaluating the mucoadhesive efficacy of the composition according to the invention on reconstructed human vaginal mucosa tissues (STERLAB Vaginal Epithelium (Batch No. 2307VAG01),) occurs through the evaluation of the percentage of inhibition of the lectin-glycoprotein bond.

Specifically, the tissues were treated with a biotinylated lectin from Canavalia ensiformis (Concanavalin A - ConA), a protein that has a high affinity for the glycosidic and mannosidic residues of membrane glycoproteins. In the absence of mucoadhesive substances, all the glycoprotein sites of the mucosal membranes are engaged in the interaction with the biotinylated lectin. Then, taking advantage of the high affinity between biotin and streptavidin, the tissues were added with streptavidin peroxidase allowing the formation of the "protein-glucose-lectin-biotin-streptavidin peroxidase" complex. The final treatment with tetramethylbenzidine (TMB), a chromogenic substrate of peroxidase that turns blue and then yellow under acidic conditions, allows the quantification of the number of lectin-biotinylated glycoprotein sites by exploiting the absorbance (ABS) at 450 nm (detectable with a microplate reader).

The intensity of the staining is in fact proportional to the quantity of glycoprotein-lectin bonds present and therefore to the quantity of available sites (glycoproteins). In the absence of preliminary treatment of the tissue with the mucoadhesive composition according to the invention, all the mucosal glycoprotein sites will be available to interact with ConA and the absorbance value thus determined will coincide with the control.

In determining the mucoadhesiveness of a product, the tissues are treated beforehand with the composition under examination; if the latter contains mucoadhesive substances, these will bind to the glycosidic and mannosidic sites present in the membrane glycoproteins. In the next phase, by adding the sequence of reagents that leads to the formation of the immune complex, a less intense staining will be obtained compared to the control due to the fact that a part of the glycosidic sites available for binding with Con-A have already been occupied by the mucoadhesive substances present in the tested sample. Therefore, the decrease in the absorbance value will be proportional to the ability of the substances under examination to mucoadhere to the mucosal cells.

The mucoadhesive capacity is expressed as a percentage of inhibition of the glycoprotein-lectin bond or better as a percentage of mucoadhesion of the product according to the following formula:

$$\%mucoadhesion = (1 - ABS\ sample\ /\ ABS\ control) \times 100$$

A sample that presents absorbance values significantly different from the negative control ($p < 0.05$, T-test for independent data) is considered mucoadhesive. A statistical comparison was also made between the different experimental conditions to evaluate any significant differences (T-test for independent data).

Internal quality controls were performed on all tissues to ensure the suitability of the biological model and verify the absence of pathologies to guarantee the safety of use for operators.

- reconstructed vaginal mucosa treated with the sample VAGINAL GEL - CODE: S03101 LOT RS52123

- vaginal mucosa reconstructed without any treatment, UNTREATED CONTROL, CTR-

The experimental model used in this test is represented by STERLAB Vaginal Epithelium (Batch N° 2307VAG01), i.e. 0.5 cm$^2$ of vaginal epithelium reconstructed starting from the A431 cell line in culture on a polycarbonate filter for 5 days in a defined medium and at an airlliquid interface. The tissues were maintained with a specific culture medium (Sterlab - batch N° 23HC136) until the end of the test in standard conditions (37°C, 5% CO2, 95% RH).

The sample was tested as is, without preliminary preparations or dilutions, in the form of a gel.

As a control condition, tissues treated only with phosphate buffer were used.

For the test, reconstructed human vaginal mucosa tissues were treated with the test product. Incubation was continued for 10 hours at 37°C. At the end of the treatment, the tissues were washed three times with saline solution to remove the non-adherent sample. The tissues were then incubated with 10 mg/L of biotinylated Con-A with specific antibodies at 37°C for 30 minutes to form the immune complex, washed three times with saline solution and then incubated for 30 minutes at 37°C with streptavidin peroxidase with gentle agitation. After 3 washes with saline solution to avoid binding interference, the tissues were treated with tetramethylbenzidine (TMB) and the absorption at 450 nm was evaluated using a microplate reader.

Three replicates of each experimental condition (treated and control) were set up.

Table 1 below shows the results obtained in this study. The results are reported as mean absorbance (ABS, ± st.dev.) and % mucoadhesiveness.

Data significant compared to the negative control (CTR-) are reported with an asterisk (*).

Table 1

| Treatment | ABS | %mucoadhesion |
|-----------|-----|---------------|
| CTR- | 3.047 ± 0.375 | - |

(continued)

| Treatment | ABS | %mucoadhesion |
|---|---|---|
| Vaginal gel | 0.082 ± 0.033 | 97.3 |

**Evaluation of film-forming capacity.**

For the in vitro instrumental evaluation of the film-forming potential of the composition according to the present invention, a Franz diffusion cell (PERMEGEAR) was used, having a diameter of 20 mm and a 10 ml receiving chamber with a flat bottom, transparent glass walls, a stirrer and a clamp (#4G-01-00-20-10). GE Healthcare Life Sciences Whatman 5 filter paper was used to perform the test.

A weighed sample of vaginal gel was applied and evenly distributed on the paper filter, inserted into the Franz diffusion cell between the donor chamber and the receiving chamber. Subsequently, a known volume of 0.5% Trypan Blue was applied through the donor chamber and the system was then incubated at 37°C. Samples were taken from the receiving chamber 8h and 10h after the start of the test for spectrophotometric evaluation of the possible presence of Trypan Blue (instrumental readings at 540 nm). Three independent tests were performed and for each experimental time evaluated the results were statistically compared with T0, i.e. the "time zero of the test start" (T-test for paired data).

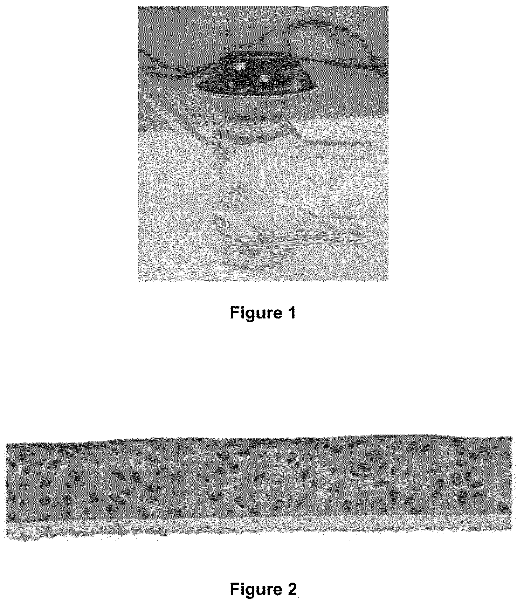

500 mg of vaginal gel (CODE: S03101 LOT. RS52123, composition reported above) were applied and evenly distributed on the paper filter, inserted in the Franz diffusion cell between the donor chamber and the receiving chamber. Subsequently, 500 $\mu$l of Trypan Blue 0.5% were applied through the donor chamber and immediately distributed over the entire available surface. The system was then placed at 37°C. Samples were taken from the receiving chamber after 8h and 10h from the start of the test (Table 2). Up to 10h from the start of the test, Trypan Blue did not permeate the substrate in the area where the product had been previously applied. (Figure 1)

Table 2

| | OD1 | OD2 | OD3 | average | st.dev. | T-test vs T0 |
|---|---|---|---|---|---|---|
| T0 | 0.041 | 0.040 | 0.039 | 0.040 | 0.001 | |
| 8h | 0.046 | 0.043 | 0.039 | 0.043 | 0.004 | 0.208 |
| 10am | 0.044 | 0.041 | 0.040 | 0.042 | 0.002 | 0.130 |

Absorbance (OD) values of the 0.5% Trypan Blue solution permeated over time (T0, 8h and 10h) in the receiving chamber.

Spectrophotometric analysis of the solution taken from the receiving chamber during the period of application of the 0.5% Trypan Blue solution on the filter treated with the product did not show significant variations up to 10 hours after the start of the test.

The tested product therefore showed film-forming properties.

**Evaluation of cytotoxicity.**

For the determination of cytotoxicity, the vaginal gel (CODE: S03101 LOT. RS52123) was used, the composition of which is reported above.

The cytotoxic effect of the composition according to the invention on cell cultures is measured by means of a cell viability test with Neutral Red Uptake (NRU). The NRU test measures cell viability, based on the cell's ability to incorporate the Neutral red dye.

The adsorption vehicle used as an inert matrix is tested in parallel with the sample to verify non-specific cytotoxic effects not due to the tested product.

Positive controls, comprised in each test as quality control and prepared with the same procedure as the samples, are made with Sodium Dodecyl Sulfate (SDS, Sigma Aldrich, Cod. 436143, Lot MKCQ6608) at various 1:2 scalar concentrations.

The product under examination is tested as is by direct contact for adsorption on an inert matrix. The product is collected inside a sterile test tube and subsequently an inert matrix of the size of approximately 1/10 of the surface of the well is soaked in the product.

The cells chosen as a cellular model (BALB/c 3T3, clone 31, ATCC CCL-163) are seeded and exposed to the tested product as is or to an extract thereof in a range of concentrations. In this specific case, the sample was tested as is adsorbed on an inert matrix. The control relating to the inert matrix only was comprised in the test under the name of "vehicle". After 24 hours of incubation, the absorption (uptake) of Neutral Red in the lysosomes/endosomes and in the vacuoles of living cells is used as a quantitative indicator of the number of cells and cell viability.

The evaluation of the cytotoxic effect of the test product is performed by comparing the 70% optical density (OD) value of the negative control (and corresponding to 70% cell viability) with the mean OD value of the test sample $\pm$ IC 95%.

If after contact with the composition of the present invention tested as is or at the highest concentration of extract (100%) the mean OD - IC95% value is $\geq$ 70%, the sample is classified as "non-cytotoxic".

If after contact with the composition of the present invention tested as is or at the highest concentration of extract (100%) the mean value of OD + IC95% is < 70%, the sample is classified as "potentially cytotoxic".

Where possible, the $IC_{50}$ value (product concentration causing a 50% reduction in cell viability) is calculated.

Negative/Blank Control (BK): The absolute optical density value (OD540 of NRU) obtained in untreated cells must be $\geq$ 0.3 (Quality check OD). To ensure uniform plating of cells, two rows of controls must be plated at the ends of the 96-well plate and the mean OD of the two rows must not differ by more than 15% from the mean of all blanks. The value found has been named "Quality check Diff". Additionally, each plate is examined under phase contrast microscopy to ensure that the quantity of cells is consistent.

Positive control: The IC50 value of the SDS must be within the 95% confidence interval (95% CI) of the historical mean (0.093 mg/mL), equal to 0.070-0.116 mg/mL (Quality check 95% CI).

Concentration-response: the IC50 derived from the dose-response curve must be supported by at least 2 or 3 responses between 10% and 90% NRU inhibition.

To determine the potential cytotoxicity of the composition, the following were used:

- BALB/c 3T3 cell line, clone 31 (ATCC CCL-163). It was used as culture medium:

  - Dulbecco's Modified Eagle's Medium (DMEM) high glucose (BIOWEST Cod.L0104 Lot. MS01QW} culture medium, added with 10% (for the maintenance medium) or 5% (for the test medium) of newborn calf serum (BCS, SIGMA ALDRICH COD 12138C LOT 20J248), 100 IU/ml Penicillin-100 $\mu$g/ml Streptomycin (SIGMA ALDRICH COD P4333 LOT 0000205653) and 1% HEPES solution (SIGMA ALDRICH COD H0887 LOT RNBK9374).

Operating procedure:

DAY 1: A suspension of BALB/c 3T3 cells, clone 31, is seeded into 96-well plates at a density of 1 x 104 cells/well and maintained in culture for 24h (5% CO2, 37°C, humidity > 90%).

DAY 2: After 24 h of incubation, the culture medium is gently aspirated from each well and replaced with new medium containing the composition according to the invention, positive controls, negative controls and vehicle. The cells are incubated again for 24 h (5% CO2, 37°C, humidity > 90%).

DAY 3: Cells are observed under the microscope for a first visual assessment, then the culture medium, samples and controls are removed. Cells are washed in PBS, stained with a Neutral Red (NR) solution and incubated for 3h (5% CO2, 37°C, humidity > 90%). At the end of the incubation period, the medium containing the dye is removed and the cells are washed with PBS. A solubilizing solution (50% ETOH/1% acetic acid/49% deionized water) is added to each well, the plates are shaken for at least 10 minutes and then read on a plate reader at 540 nm.

The negative control (NC) showed a mean $OD_{540}$ value of 0.693 *(Quality check OD > 0.3)* and was also found to be compliant with the *Quality check Diff.*

The positive control (PC) with sodium dodecyl sulfate showed a mean $IC_{50}$ value of 0.078 $\pm$ 0.003 *(Quality check IC 95% compliant).*

The vaginal gel according to the invention showed a cell viability of 38.42% and was therefore potentially cytotoxic.

The formulation has a low pH and contains lactic acid which, in itself, is not cytotoxic in vivo. It is a normal by-product of anaerobic metabolism that occurs in our bodies during intense exercise or in some pathological conditions. However, in an in vitro test, where cells are grown in the laboratory and normally maintained at a pH of 7.0 to 7.6, lactic acid can have cytotoxic effects due to the acidification of the cell culture medium. This acidic shift in pH can be harmful to cells, as it disrupts normal cell functions and can lead to cell death.

In addition, lactic acid can affect the osmotic balance of cells. An accumulation of lactic acid can alter the normal balance of ions and molecules within the cell, causing osmotic stress. This stress can cause cell contraction, dysfunction and ultimately cell death, therefore in vitro cytotoxicity does not translate into adverse effects on the vaginal mucosa in clinical use.

**Assessment of irritation potential.**

To determine the potential irritant effects of the composition according to the invention on the vaginal mucosa, the vaginal gel (CODE: S03101 LOT. RS52123) was used, the composition of which is reported above.

The three-dimensional cellular model used (EpiSkin, Lyon, Lot 23-HVE-018) consists of $0.5\,cm^2$ diameter units of transformed human vaginal epithelial cell cultures, maintained for 5 days in chemically defined culture medium and in contact with air on inert polycarbonate filters.

Figure 2 shows a 3D vaginal epithelium model (Skinethic®) grown in vitro. A multilayered epithelium without stratum corneum resting on a basement membrane can be recognized. The product to be tested is applied to the epithelium, underneath which there is a semi-permeable membrane that communicates with the lower well where the culture medium is located.

30 µl of the composition according to the invention (as is) and controls were applied to each epithelial sample, in duplicate. For the positive control (CP) SLS (Sodium Lauryl Sulfate) dissolved at 0.5% in water was used, while cells treated with water only were used as negative control (CN).

Exposure was carried out for 4 and 24 hours at 37°C, 5% $CO_2$. At the end of exposure, washing was performed with PBS to remove the product and evaluate cell viability using the MTT test.

The cell viability test (MTT) was performed by incubating the epithelium for $3\pm0.5h$ at 37°C with an MTT solution (3-[4,5-dimethylthiazol-2-yl]-2,5-diphenyl tetrazolium bromide). Then the solution was removed and replaced with isopropanol with subsequent incubation at room temperature (1.5-2h) or at 4°C (overnight). Two aliquots of each sample were then transferred to a plate with wells for absorbance reading (570 nm) by spectrophotometer equipped with a plate reader, subtracting the background reading at 690 nm. The absorbances were corrected by subtracting the readings due to the blank.

The result is expressed as cell viability in percentage according to the formula:

$$\%Cell\ Viability = [OD(570\text{-}690)_{sample} / OD(570\text{-}690)_{CN}]\ x\ 100$$

$$\%\ cell\ mortality = 100 - \%\ cell\ viability$$

The cytotoxicity data obtained with the MTT assay are plotted against the exposure times, creating a dose-response curve, which allows to determine the theoretical value of $ET_{50}$ or the time in minutes that induces a 50% reduction in cell viability in the epithelium after exposure to a compound compared to untreated cells.

Method acceptance criteria:

- For negative control (CN): mean OD value of replicates must be $\geq 0.7$ and $\leq 2.5$ and standard deviation must be $\leq 20\%$
- For positive control (PC): standard deviation must be $\leq 20\%$
- for the sample: the standard deviation must be $\leq 20\%$

A reduction in cell viability > 30% is considered a cytotoxic effect. Qualitative interpretation of the result is performed by comparing the $ET_{50}$ of the sample with that of a mild irritant, 0.5% SLS.

From the tests conducted, the results obtained were in accordance with the acceptability criteria reported in Table 3 below making the test for the evaluation of irritation valid.

Table 3

| | Value | | Limits | Result |
|---|---|---|---|---|
| | 4h | 24h | | |
| OD$_{(CN)}$ average 2 replicates | 1.07 | 0.77 | $\geq 0.7 - \leq 2.5$ | Compliant |
| CN Std. Dev. % | 4.04 | 10.13 | $\leq 20\%$ | Compliant |
| CP Std. Dev. % | 6.26 | 0.04 | $\leq 20\%$ | Compliant |
| Sample: Std. Dev. % | 8.85 | 0.13 | $\leq 20\%$ | Compliant |

On the basis of the results provided (Table 3, 4 and Figure 3), taking into account the methods of use and the persistence of the device as is in the vaginal area, also evaluating the behaviour of a slightly irritating substance such as 0.5% SLS, it can be stated that the composition according to the invention has not shown irritating effects on the human vaginal epithelium under normal conditions of use.

Table 4

| Sample | %average cell viability (4h) | %average cell viability (24h) | ET$_{50}$ (minutes) |
|---|---|---|---|
| Vaginal gel | 90.28 ($\pm$8.85) | 3.71 ($\pm$0.13) CYTOTOXIC | 13 hours (estimated) |
| | NON-CYTOTOXIC | | Non-irritating |
| CP | 8.28 ($\pm$6.26) CYTOTOXIC | 0.52 ($\pm$0.04) CYTOTOXIC | <4h (Estimated) |
| CN | 100.00 ($\pm$4.04) | 100.00 ($\pm$10.13) | Non-irritating |

**Evaluation of sensitizing power.**

For the determination of the sensitizing power of the composition according to the invention on the vaginal mucosa, the vaginal gel (CODE: S03101 LOT. RS52123) was used, the composition of which is reported above.

The test was conducted in accordance with the method described in OECD 442E [1] and in protocol 158 EURL -ECVAM (European Union Reference Laboratory for alternatives to animal testing) [2].

A human monocytoid cell line, named THP-1 (ATCC TIB-202), was used. Cells were cultured in RPMI containing 10% FBS, 0.05 mM 2-mercaptoethanol, 100 units/ml penicillin, and 100 $\mu$g/ml streptomycin.

A preliminary cellular reactivity test was performed with DNCB and nickel sulphate as positive controls and lactic acid as negative control, two weeks after thawing the cell line (THP-1 (ATCC TIB-202),). Both positive controls produced an increase in the expression of CD86 and CD54, while lactic acid produced a negative response on the expression of the markers. At this point the cells were left in culture for up to approximately two months (the number of passages must not exceed 30).

THP1 cells were plated at a concentration of 1.6X105 cells/well in a plate with 96 wells and mixed 1:1 with the sample (the composition according to the present invention) and controls. The sample is a gel and was solubilized in saline; then, 8 stock solutions were prepared (by serial dilutions 1:2). These solutions were further diluted 50-fold in medium and then 2-fold with the cell suspension to obtain the concentrations to be tested on the cells (39.1 $\mu$g/ml; 78.1 $\mu$g/ml; 156.2 $\mu$g/ml; 312.5 $\mu$g/ml; 625.0 $\mu$g/ml; 1249.9 $\mu$g/ml; 2499.8 $\mu$g/ml; 4999.7 $\mu$g/ml).

As a negative control, culture medium was used. After 24h of exposure, cells were centrifuged and resuspended in FACS buffer and in the presence of propidium iodide (PI) for flow cytometric analysis. From here, the concentration causing 25% mortality (CV75) was determined to be used for the final test. (*range finding*)

Following the *range finding results*, the sample was solubilized in saline at a concentration corresponding to the 100X concentration of 1.2 X CV75. Then 7 stock solutions (serial dilutions 1:1.2) were prepared in saline (up to 0.335 X CV75).

The stock solutions were then diluted 50-fold in culture medium. These solutions were tested directly on cells with a further 1:2 dilution (96.8 $\mu$g/ml; 116.2 $\mu$g/ml; 139.4 $\mu$g/ml; 167.3 $\mu$g/ml; 200.8 $\mu$g/ml; 241.0 $\mu$g/ml; 289.1 $\mu$g/ml; 347.0 $\mu$g/ml).

As a negative control, culture medium was used, as a positive control, DNCB at a concentration of 4$\mu$g/ml was used. Cells were exposed to the samples for 24 h at 37°C with 5% $CO_2$. The experiment was performed in three replicates and was repeated 3 times on different days.

After exposure to the test substance, the cells were centrifuged and resuspended in FACS buffer and divided into three aliquots. After a subsequent centrifugation, the cells were resuspended in blocking solution (FACS buffer containing 0.01% gamma-globulins) and incubated for 15' at 4°C. Then, after a subsequent centrifugation, the cells were labeled with fluorescent antibodies directed against CD86, CD54 or IgG1 (as a control) for 30' at 4°C. After washing with FACS buffer, the cells were resuspended in the same in the presence of propidium iodide (PI). Then the expression of CD86, CD54 levels and cell viability were analyzed by flow cytometry.

Expression of results: Cell viability with propidium iodide staining is calculated as:

$$\text{Cell Viability:} \quad \frac{\text{total number of living cells}}{\text{total number of acquired cells}} \quad X \quad 100$$

The value of CV75, i.e. the concentration that causes 25% cell mortality or 75% cell viability in THP-1 cells is calculated by linear logarithmic interpolation according to the following formula:

$$\text{Log CV75} = \frac{(75 - B) \times \text{Log}(C) - (75 - A) \times \text{Log}(D)}{A - B}$$

where

A is the cell viability immediately greater than 75%.
B is the cell viability immediately below 75%.
C or D is the concentration that shows the cell viability values of A or B, respectively.

The CV75 value is used to calculate the concentrations to be tested in the final assay (CD86 and CD54 expression).

The expression of CD86 and CD54 is evaluated by flow cytometry. Starting from the MFI (Mean Fluorescence Intensity), which is proportional to the number of labeled molecules per cell, the RFI (relative fluorescence intensity) of CD86 and CD54 is calculated, respectively, for cells treated with the positive control and for cells treated with the sample, according to the following formula:

$$\text{RFI} = \frac{\text{MFI of sample treated cells} - \text{MFI control isotype treated}}{\text{MFI sample solvent treated cells} - \text{MFI isotype solvent treated cells}}$$

Cell viability of cells treated with the control isotype is also calculated.

Interpretation of results: if the CD86 RFI value is equal to or greater than 150% in at least one of the tested concentrations (with a corresponding cell viability > 50%) in at least two independent experiments and/or the CD54 RFI value is equal to or greater than 200% in at least one of the tested concentrations (with a corresponding cell viability > 50%) in at least two independent experiments, the prediction of sensitization is considered positive, otherwise negative. In case two different experiments do not give concordant results, a third experiment must be performed for a correct prediction.

For a substance to be considered sensitizing, the two concentrations with a positive effect, i.e. EC150 for CD86 and EC200 for CD54, i.e. the concentrations inducing an RFI of 150 or 200, respectively, must be extrapolated.

$$\text{EC150 (per CD86)} = B_{dose} + [(150 - B_{RFI}) / (A_{RFI} - B_{RFI}) \times (A_{dose} - B_{dose})]$$

$$\text{EC200 (per CD54)} = B_{dose} + [(200 - B_{RFI}) / (A_{RFI} - B_{RFI}) \times (A_{dose} - B_{dose})]$$

$A_{dose}$ = is the lowest concentration in $\mu$g/ml with RFI > 150 (CD86) or 200 (CD54).
$B_{dose}$ = is the highest concentration in $\mu$g/ml with RFI < 150 (CD86) or 200 (CD54).
$A_{RFI}$ = is the lowest concentration with RFI > 150 (CD86) or 200 (CD54).
$B_{RFI}$ = is the highest concentration with RFI < 150 (CD86) or 200 (CD54).

In order to measure EC150 and EC200 more accurately, three experiments should be done. The EC150 and EC200 values are the average of the values calculated for each experiment. When only two of three different experiments have positive results, the highest value of EC150 and EC200 is considered.

Acceptance criteria:

- The cell viability of the control medium and solvent must be > 90%,
- In the DNCB positive control, the RFI values of both CD86 and CD54 markers should be > 150 and > 200, respectively, and the cell viability > 50%,
- In the DMSO solvent control (if used), the RFI values of both markers (CD86 and CD54) must not exceed the positivity values (150 and 200 for CD86 and CD54 respectively),
- In the control medium and DMSO (if used) the MFI % value of both markers in the control isotype must be > 105%,
- Cell viability of the sample tested for more than 4 doses must be > 50% for each experiment.

Negative results are acceptable only for those substances showing a cell viability at the concentration 1.2 X CV75 lower than 90%. If the cell viability is higher than or equal to 90% the results would not be acceptable and the *dose finding range* should be repeated.

Positive results are acceptable for any cell viability shown at the concentration 1.2 X CV75.

When 1000 μg/ml in DMSO or 5000 μg/ml in PBS or the highest soluble concentration is used as the highest concentration, the results are acceptable.

As can be seen from the following Table 5, the test results are in compliance with the test acceptability criteria and therefore the test was considered valid.

Table 5

| | **Limiti/ Limits** | **Risultato/Result** |
|---|---|---|
| Vitalità cellulare del medium/ Cell viability of medium | >90% | Experiment 1: 91%; 91%; 90%<br>Experiment 2: 90%; 90%; 90%<br>Experiment 3: 90%; 91%; 90% |
| RFI DNCB | >150 (CD86)<br>>200 (CD54) | Experiment 1: 489 (CD86); 232 (CD54)<br>Experiment 2: 493 (CD86); 229 (CD54)<br>Experiment 3: 479 (CD86); 226 (CD54) |
| MFI% medium / DMSO | >105% | Experiment 1: +135 % (CD86); +140 % (CD54)<br>Experiment 2: +135 % (CD86); +140 % (CD54)<br>Experiment 3: +136 % (CD86); +140 % (CD54) |
| Vitalità cellulare del campione/ Cell viability of sample | per più di 4 dosi deve essere > 50%/ > 50% viability in more than four tested doses | Experiment 1: 8 doses > 50%<br>Experiment 2: 8 doses > 50%<br>Experiment 3: 8 doses > 50% |

The following are the results of the study of cell viability as a function of the dose (table 6, Figure 4) and of the expression of the signal substances CD86 and CD54 by determining the RFI%. (table 7)

Table 6

| | **Dose (μg/ml)** | **39.1** | **78.1** | **156.2** | **312.5** | **625.0** | **1249.9** | **2499.8** | **4999.7** |
|---|---|---|---|---|---|---|---|---|---|
| RUN 1 | **Viability %** | 77.9 | 76.1 | 75.7 | 73.5 | 68.6 | 63.5 | 63.2 | 60.3 |
| RUN 2 | **Viability %** | 78.8 | 77.2 | 77.1 | 76.9 | 70.4 | 65.2 | 64.1 | 59.4 |

| | **RUN 1** | **RUN 2** | | |
|---|---|---|---|---|
| **Dose (μg/ml)** | **312.5** | **625.0** | | **194.7** |
| **Viability %** | 73.5 | 70.4 | < 75 | |
| **Dose (μg/ml)** | **156.2** | **312.5** | | **383.4** |
| **Viability %** | 75.7 | 76.9 | >75 | ***AVERAGE 289.1*** |

Table 7

| **Vaginal Gel S03101**<br>***Lotto/Batch: RS52123*** | **ESPERIMENTO 1/ RUN1** | | **ESPERIMENTO 2/ RUN2** | | **ESPERIMENTO 3/ RUN3** | |
|---|---|---|---|---|---|---|
| | **RFI %**<br>**(CD86)** | **RFI %**<br>**(CD54)** | **RFI %**<br>**(CD86)** | **RFI %**<br>**(CD54)** | **RFI %**<br>**(CD86)** | **RFI %**<br>**(CD54)** |
| Controllo negativo/ Negative control MEDIUM | 100 | 100 | 100 | 100 | 100 | 100 |
| Controllo positivo/ Positive control DNCB 4μg/ml | 489 | 232 | 493 | 229 | 479 | 226 |
| 96.8 μg/ml | 85 | 92 | 86 | 93 | 84 | 92 |
| 116.2 μg/ml | 106 | 93 | 108 | 93 | 104 | 92 |
| 139.4 μg/ml | 100 | 87 | 102 | 87 | 98 | 85 |
| 167.3 μg/ml | 74 | 97 | 75 | 97 | 72 | 96 |

(continued)

| Vaginal Gel S03101 Lotto/Batch: RS52123 | ESPERIMENTO 1/ RUN1 | | ESPERIMENTO 2/ RUN2 | | ESPERIMENTO 3/ RUN3 | |
|---|---|---|---|---|---|---|
| | RFI % (CD86) | RFI % (CD54) | RFI % (CD86) | RFI % (CD54) | RFI % (CD86) | RFI % (CD54) |
| 200.8 µg/ml | 81 | 102 | 82 | 102 | 80 | 101 |
| 241.0 µg/ml | 92 | 108 | 94 | 108 | 91 | 107 |
| 289.1 µg/ml | 83 | 103 | 84 | 103 | 81 | 102 |
| 347.0 µg/ml | 67 | 118 | 68 | 118 | 75 | 117 |
| CUT OFF | 150 | 200 | 150 | 200 | 150 | 200 |

Note : Each RFI value reported in the table is the average of the RFIs of the three experiments performed.

As can be seen from the data reported, the vaginal gel according to the present invention does not show a sensitizing potential.

**Evaluation of the efficiency of the preservative system.**

To determine the effectiveness of the preservative system of the composition according to the invention on the vaginal mucosa, the vaginal gel (CODE: S03101 LOT. RS52123) whose composition is reported above was used as a non-limiting example.

The strains used to study the preservation potential of the composition according to the present invention have been described in Table 8 below.

Table 8

| CEPPO STRAIN | Terreno di crescita Growth Medium | Concentrazione Inoculo Inoculum concentration (CFU/g) |
|---|---|---|
| *Pseudomonas aeruginosa* ATCC 9027 | Casein soya bean digest agar | $7,0 \times 10^5$ |
| *Staphylococcus aureus* ATCC 6538 | Casein soya bean digest agar | $1,8 \times 10^5$ |
| *Candida albicans* ATCC 10231 | Sabouraud-glucose agar | $1,2 \times 10^5$ |
| *Aspergillus brasiliensis* ATCC 16404 | Sabouraud-glucose agar | $1,0 \times 10^5$ |

The inoculum was prepared by growing the bacteria on "Casein soya bean digest agar" and the fungi on "Sabouraud glucose agar", the bacteria were incubated at $32.5 \pm 2.5°C$ for 18-24h, Candida albicans at $22.5 \pm 2.5°C$ for 2-3 days and Aspergillus brasiliensis at $22.5 \pm 2.5°C$ for 5-7 days. The concentration of the inoculum was prepared in a physiological solution with a microbial load of approximately 108 CFU/ml; the final concentration of the inoculum in the product will be between 105 and 106 CFU/ml.

The treated packages were then stored at room temperature until the next seeding. The concentration of viable cells was determined by the plate count method, diluting the product in a sodium chloride-peptone solution at pH 7 with the addition of some preservative neutralizing agents (polysorbate 80, soy lecithin, L-Histidine). The counting of microorganisms at different times was carried out by diluting 1 g or ml of product up to 1x106 times and plating each dilution in petri dishes containing the agar culture media (see previous Table).

The plates were incubated at $32.5 \pm 2.5°C$ (bacteria) or at $22.5 \pm 2.5°C$ (yeasts and moulds) for the time necessary to allow good growth of the microorganism (time ≤ 3 days for bacteria and ≤ 5 days for yeasts and moulds). By correcting the number of colonies observed for the dilution factor, the value of CFU (Colony Forming Units) per gram or milliliter of product is obtained. Sowings are carried out at 2, 7, 14 and 28 days from inoculation in order to evaluate the dynamics of microbial reduction.

The efficacy of the preservative system is considered adequate for a topical preparation, according to the more restrictive "A" criteria, when the challenge shows a reduction in microbial growth of at least 2 logs after 2 days, of at least 3 logs after 7 days for bacteria without any subsequent regrowth on day 28 and of at least 2 logs, for fungi, after 14 days without any subsequent regrowth on day 28, as summarised in the following Table 9:

Table 9

| | | Riduzione in log *Log reduction* | | | |
|---|---|---|---|---|---|
| | | 299 | 7gg | 14gg | 28gg |
| Batteri *Bacteria* | A | 2 | 3 | --- | NI |
| | B | --- | --- | 3 | NI |
| Funghi *Fungi* | A | --- | --- | 2 | NI |
| | B | --- | --- | 1 | NI |
| NI: No increase compared to the previous time | | | | | |

The less restrictive B criteria may be applied in particular justified cases where the A criteria cannot be met, for example to avoid an increased risk of adverse reactions.

Below, in Table 10, the total microbial count is reported while the reduction of microbial growth (in logarithmic scale) is described in Table 11.

Table 10

| Tempo | *Pseudomonas aeruginosa CFU/g* | *Staphylococcus aureus CFU/g* | *Candida albicans CFU/g* | *Aspergillus brasiliensis CFU/g* |
|---|---|---|---|---|
| Tinocaum | $7,0 \times 10^5$ | $1,8 \times 10^5$ | $1,2 \times 10^5$ | $1,0 \times 10^5$ |
| 2 days | <10 | <10 | $9,0 \times 10^3$ | $2.1 \times 10^4$ |
| 7 days | <10 | <10 | <10 | $1,6 \times 10^2$ |
| 14 days | <10 | <10 | <10 | <10 |
| 28 days | <10 | <10 | <10 | <10 |

Table 11

| Tempo | *Pseudomonas aeruginosa* | *Staphylococcus aureus* | *Candida albicans* | *Aspergillus brasiliensis* |
|---|---|---|---|---|
| 2 days | > 4,85 | > 4,26 | 1,12 | 0,68 |
| 7 days | > 4,85 | > 4,26 | > 4,08 | 2,80 |
| 14 days | > 4,85 | > 4,26 | > 4,08 | > 4,00 |
| 28 days | > 4,85 e NI | > 4,26 e NI | > 4,08 e NI | > 4.00 e NI |

Based on the results expressed in Table 12 shown below, the vaginal gel according to the present invention satisfies, according to criteria A, the requirements of the test for the evaluation of the efficiency of the preservative system.

Table 12

| CEPPO / STRAIN | *Non soddisfa i criteri / Does not satisfy the criteria* | *Soddisfa i criteri A / Satisfies criteria A* | *Soddisfa i criteri B / Satisfies criteria B* |
|---|---|---|---|
| *Pseudomonas aeruginosa* ATCC 9027 | | X | |
| *Staphylococcus aureus* ATCC 6538 | | X | |
| *Candida albicans* ATCC 10231 | | X | |
| *Aspergillus brasiliensis* ATCC 16404 | | X | |

**Test for the evaluation of the efficiency of the preservative system during the use of the finished product.**

To determine the efficiency of the preservative system of the finished product during its use on the vaginal mucosa, the vaginal gel (CODE: S03101 LOT. RS52123), the composition of which is reported above, was used by way of non-limiting example.

The use test described was developed to simulate what happens with the normal use of finished products, in compliance with a defined laboratory protocol. The test was conducted on the vaginal gel (applied through a cannula) simulating the use by the consumer for a total of 30 days and introducing microbial contamination with some strains (Escherichia coli, Staphylococcus aureus and Candida albicans) by swabbing with a cotton swab, and performing the cannula washing procedure as described below.

The inoculation is carried out with a mixture of the microbial strains reported in Table 13 at the indicated concentrations:

Table 13

| CEPPO STRAIN | Terreno di crescita Growth Medium | Concentrazione Inoculo Inoculum concentration (CFU/p) |
|---|---|---|
| *Escherichia coli* ATCC 8739 | Violet Red Bile agar | $1,8 \times 10^3$ |
| *Staphylococcus aureus* ATCC 6538 | Mannitol Salt Agar | $2,1 \times 10^3$ |
| *Candida albicans* ATCC 10231 | Sabouraud-destrose agar | $2,3 \times 10^3$ |

During the in-use stability test described here, the cannula used to dispense the product to the consumer was subjected to repeated inoculations with 100 µl of the above-mentioned microorganisms by *swabbing* with a cotton swab, after simulating use and then dispensing 5 grams of product. After contamination, the cannula was washed, left to dry under a hood, reassembled and placed in a clean closed jar until the next simulation of use.

The product was then stored for the entire duration of the test at room temperature. At different times, the percentage of reduction of the microbial load was evaluated through analysis on selective media specific for the 3 inoculated microorganisms and an opinion was formulated on the resistance of the product to the simulated use test. The inoculation was repeated 3 days a week for 30 days. At different analysis times (at T8, 15, 22 and 30 days) the microorganism count was carried out by taking 5 g of product and diluting them 1:10 in neutralizing solution (consisting of Tween 80, soy lecithin, L-histidine, sodium thiosulfate, sodium chloride, potassium dihydrogen phosphate, disodium phosphate, peptone, water). Each dilution was then plated on Petri dishes containing the selective agar culture medium.

The plates were incubated at 32.5°C ± 2.5°C for the time necessary to allow sufficient growth of the microorganism for counting. By correcting the number of colonies observed for the dilution factor, it was possible to obtain the value of CFU (Colony Forming Units) per gram of product. The test was performed in duplicate.

Below are the instructions for using the medical device and washing it:

1. Wash your hands thoroughly before and after using the medical device;

2. Pierce the aluminum gasket of the tube using the wedge on the cap;

3. Push the cannula piston all the way down to completely expel the air inside and screw it into the tube;

4. Squeeze the tube until the cannula is full;

5. Unscrew the cannula from the tube and close the tube with the cap;

6. To apply the gel, lie down, insert the tip of the cannula into the vagina and slowly squeeze the tube again to release the gel;

7. The product must be used by one person only. After use, disassemble the cannula, remove the piston and wash both thoroughly with warm water (do not immerse in boiling water) and soap or neutral intimate detergent. Finally, rinse, taking care not to leave soap residue. Leave them to dry in a clean place.

Reassemble the piston inside the cannula only after complete drying. Store inside the box until next use, following the storage instructions indicated on the leaflet.

The device used to perform the test is an applicator that has a threaded end to attach to the vaginal gel tube and a plunger that moves back when the cannula is filled with the product. Once the cannula is filled, it is unscrewed from the tube and can be used by returning the plunger to its initial position with light pressure.

Below, in Table 14, is the protocol for the stability test in use:

Table 14

| Giorni/Days | Activities |
|---|---|
| T1 | Open and assemble the cannula. Execute a preliminary count of the product. Contaminate the cannula, wash it, let it dry and close the tube again. |
| T3 | Open and assemble the cannula. Simulate the use of the product by dispensing 5g. |
| T5 | |
| T10 | |
| T12 | |
| T17 | |
| T19 | Contaminate the cannula, wash it, let it dry, and close the tube again. |
| T24 | |
| T26 | |
| T8 | Open and assemble the cannula. Simulate the use of the product by dispensing 5g and carry out an analysis on selective mediums. |
| T15 | |
| T22 | Contaminate the cannula, wash it, let it dry, and close the tube again. |
| T30 | Open and assemble the cannula. Simulate the use of the product by dispensing 5g and carry out an analysis on selective mediums. |

The wash was performed with a neutral intimate detergent.

A preliminary microbial count was performed at T 1 day on the sample under examination to ensure the absence of ongoing contamination. (Table 15)

Table 15

| Parametro / Parameter | Metodo / Method | CFU/g o ml |
|---|---|---|
| Conta totale batteri aerobi<br>Total viable count aerobic bacteria | ISO 21149 | <10 |
| Conta totale lieviti e muffe<br>Total viable count yeast and mould | ISO 16212 | <10 |

Microbiological stability at the use test is considered optimal when the reduction is >90%, and without increases over time. The results of total microbial counts at different analysis times and of the reduction of microbial growth are reported in Tables 16, 17 respectively.

Table 16

| Tempo Time | Escherichia coli CFU/plate (*) | Staphylococcus aureus CFU /plate (*) | Candida albicans CFU /plate (*) |
|---|---|---|---|
| T0 | $1,8 \times 10^2$ | $2,1 \times 10^2$ | $2,3 \times 10^2$ |
| 8 days | 0 | 0 | 0 |
| 15 days | 0 | 0 | 0 |
| 22 days | 0 | 0 | 0 |
| 30 days | 0 | 0 | 0 |
| (*) At each analysis time the results are expressed as the average of two values. | | | |

Table 17

| Tempo Time | Escherichia coli | Staphylococcus aureus | Candida albicans |
|---|---|---|---|
| 8 days | >99,9% | >99,9% | >99,9% |
| 15 days | >99,9% | >99,9% | >99,9% |
| 22 days | >99,9% | >99,9% | >99,9% |
| 30 days | >99,9% | >99,9% | >99,9% |

From the analysis of the tables, the vaginal gel according to the invention appears to have an effective reduction of all the tested strains during the entire simulation test (30 days of use with the applicator) with the cannula washing procedure described above.

**REFERENCES**

[1] OECD GUIDELINE FOR THE TESTING OF CHEMICALS. Test Guideline No. 442E. In vitro Skin Sensitization: In Vitro Skin Sensitization assays addressing the Key Event on activation of dendritic cells on the Adverse Outcome Pathway for Skin Sensitization. 30 June 2022.

[2] DB-ALM (INVITTOX) (2014) Protocol 158: human Cell Line Activation Test (h-CLAT).

**Claims**

1.  Composition comprising:

    - at least one mucoadhesive agent in a quantity between 0.01% and 15% w/w or v/v, preferably between 0.1% and 5% w/w or v/v,
    - at least one moisturizing agent in a quantity between 0.01% and 15% w/w or v/v preferably between 0.1% and 5% w/w or v/v,
    - at least one pH regulator in a quantity between 0.01% and 10% w/w or v/v, preferably between 0.01% and 5% w/w or v/v,
    - at least one agent for the control of bacterial proliferation in a quantity between 0.001% and 8% w/w or v/v, preferably between 0.001% and 5% w/w or v/v,
    - at least one agent for the rebalancing of the vaginal bacterial flora in a quantity between 0.001% and 8% w/w or v/v, preferably between 0.001% and 5% w/w or v/v,
    - at least one diluent or solvent in a quantity sufficient to reach 100% w/w or v/v;
    wherein:

        - said composition has a pH between 3-5,
        - said at least one agent for the control of bacterial proliferation is chosen from:

            • ferulic acid in a quantity between 0.01-5% w/w or v/v;
            • resveratrol in a quantity between 0.01-5% w/w or v/v; and
            • combinations thereof;

        - said agent for the rebalancing of the bacterial flora comprises or consists of sodium carboxymethyl betaglucan in a quantity between 0.001-5% w/w or v/v.

2.  Mucoadhesive composition for vaginal use comprising:

    - sodium hyaluronate with a molecular weight preferably selected between 100-5000 kDa and mixtures thereof, in a quantity between 0.1-5% w/w or v/v;
    - glycogen in a quantity between 0.01-8% w/w or v/v;
    - lactic acid in a quantity between 0.01-5% w/w or v/v,

- sodium carboxymethyl betaglucan in a quantity between 0.001-5% w/w or v/v;
- a diluent and/or solvent in a quantity sufficient to reach 100% w/w or v/v; and
- ferulic acid in a quantity between 0.01-5% w/w or v/v, and/or resveratrol in a quantity between 0.01-5% w/w or v/v;

and wherein said composition has a pH between 3-5.

3. Composition according to any of the preceding claims in the form of a gel, preferably having a viscosity between 10,000 and 90,000 cPs measured by a Rotavisc ME-VI S000 Viscometer.

4. Composition according to any of the preceding claims, further comprising at least one ingredient selected from antioxidant agents, preservatives, pH regulators, texturizers and other pharmaceutically and/or cosmetically acceptable excipients, and mixtures thereof, preferably selected from the group of tocopherol, tocopherol acetate, lycopene, ascorbic acid, resveratrol, ferulic acid, coenzyme Q10, propanediol, polysorbate 20, 1,2-hexanediol, caprylyl glycol, tropolone, disodium phosphate, polysorbate 60, sodium phosphate.

5. Composition according to any of the preceding claims, comprising an antioxidant agent in a quantity between 0.01-5% w/w or v/v.

6. Composition according to any of the preceding claims, comprising an additive in a quantity between 0.0001% - 5% w/w or v/v.

7. Mucoadhesive composition for vaginal use comprising:

- sodium hyaluronate (with a molecular weight preferably chosen between 100-5000 kDa, even more preferably between 200-1000 kDa and mixtures thereof) in a quantity between 0.1-5% w/w or v/v;
- glycogen in a quantity between 0.01-8% w/w or v/v;
- ferulic acid in a quantity between 0.01-5% w/w or v/v,
- lactic acid in a quantity between 0.01-5% w/w or v/v,
- resveratrol in a quantity between 0.01-5% w/w or v/v,
- sodium carboxymethyl betaglucan in a quantity between 0.001-5% w/w or v/v,
- a diluent and/or solvent in a quantity sufficient to reach 100% w/w or v/v.

8. Composition according to any of the preceding claims wherein:

- sodium hyaluronate, with a molecular weight between 200-500 kDa and mixtures thereof, is present in a quantity between 0.1-2.00% w/w or v/v, preferably approximately 0.50% w/w or v/v;
- glycogen is present in a quantity between 0.04-0.80% w/w or v/v, preferably about 0.20% w/w or v/v;
- ferulic acid is present in a quantity between 0.05-0.30% w/w or v/v, preferably about 0.15% w/w or v/v;
- lactic acid is present in a quantity between 0.02-0.20% w/w or v/v, preferably about 0.09% w/w or v/v;
- resveratrol is present in a quantity between 0.05-0.30% w/w or v/v; preferably about 0.08% w/w or v/v;
- sodium carboxymethyl betaglucan is present in a quantity between 0.001-0.01% w/w or v/v, preferably about 0.0021% w/w or v/v;
- propanediol is present in a quantity between 1.00-5.00% w/w or v/v;
- polysorbate-20 is present in a quantity between 0.5-3.00% w/w or v/v;
- hydroxyethyl cellulose is present in a quantity between 0.1-2.00% w/w or v/v;
- 1,2-hexanediol is present in a quantity between 0.01-1.50% w/w or v/v;
- caprylyl glycol is present in a quantity between 0.01-1.50%w/w or v/v;
- tropolone is present in a quantity between 0.0002-0.02% w/w or v/v;
- disodium phosphate is present in a quantity between 0.0005-0.02% w/w or v/v;
- polysorbate-60 is present in a quantity between 0.0005-0.02% w/w or v/v;
- sodium phosphate is present in a quantity between 0.0001-0.01% w/w or v/v;
- water is present in a quantity necessary to reach 100% w/w or v/v.

9. Composition according to any of the preceding claims, wherein:

- sodium hyaluronate, with a molecular weight between 200-500 kDa and mixtures thereof, is present in a quantity between 0.1-2.00% w/w or v/v, preferably approximately 0.50% w/w or v/v;
- glycogen is present in a quantity between 0.04-0.80% w/w or v/v, preferably about 0.20% w/w or v/v;

- lactic acid is present in a quantity between 0.02-0.20% w/w or v/v, preferably about 0.09% w/w or v/v;
- resveratrol is present in a quantity between 0.05-0.30% w/w or v/v, preferably about 0.08% w/w or v/v;
- sodium carboxymethyl betaglucan is present in a quantity between 0.001-0.01% w/w or v/v, preferably about 0.0021% w/w or v/v;
- propanediol is present in a quantity between 1.00-5.00% w/w or v/v,
- polysorbate-20 is present in a quantity between 0.5-3.00% w/w or v/v,
- hydroxyethyl cellulose is present in a quantity between 0.1-2.00% w/w or v/v;
- 1,2-hexanediol is present in a quantity between 0.01-1.50% w/w or v/v;
- caprylyl glycol is present in a quantity between 0.01-1.50% w/w or v/v;
- tropolone is present in a quantity between 0.0002-0.02% w/w or v/v;
- disodium phosphate is present in a quantity between 0.0005-0.02% w/w or v/v;
- polysorbate-60 is present in a quantity between 0.0005-0.02% w/w or v/v;
- sodium phosphate is present in a quantity between 0.0001-0.01% w/w or v/v;
- water is present in a quantity necessary to reach 100% w/w or v/v.

10. Composition according to any of the preceding claims, wherein:

- sodium hyaluronate, with a molecular weight between 200-500 kDa and mixtures thereof, is present in a quantity between 0.1-2.00% w/w or v/v, preferably approximately 0.50% w/w or v/v;
- glycogen is present in a quantity between 0.04-0.80% w/w or v/v, preferably about 0.20% w/w or v/v;
- ferulic acid is present in a quantity between 0.05-0.30% w/w or v/v, preferably about 0.15% w/w or v/v;
- lactic acid is present in a quantity between 0.02-0.20% w/w or v/v, preferably about 0.09% w/w or v/v;
- sodium carboxymethyl betaglucan is present in a quantity between 0.001-0.01% w/w or v/v, preferably about 0.0021% w/w or v/v;
- propanediol is present in a quantity between 1.00-5.00% w/w or v/v;
- polysorbate-20 is present in a quantity between 0.5-3.00% w/w or v/v;
- hydroxyethyl cellulose is present in a quantity between 0.1-2.00% w/w or v/v;
- 1,2-hexanediol is present in a quantity between 0.01-1.50% w/w or v/v;
- caprylyl glycol is present in a quantity between 0.01-1.50% w/w or v/v;
- tropolone is present in a quantity between 0.0002-0.02% w/w or v/v;
- disodium phosphate is present in a quantity between 0.0005-0.02% w/w or v/v;
- polysorbate-60 is present in a quantity between 0.0005-0.02% w/w or v/v;
- sodium phosphate is present in a quantity between 0.0001-0.01% w/w or v/v;
- water is present in a quantity necessary to reach 100% w/w or v/v.

11. Composition according to any of the preceding claims formulated as a lotion, cream, ointment, paste, salve, ovules.

12. Composition according to any of the preceding claims for use in the treatment of vaginitis, vaginosis, vaginal atrophy or atrophic vaginitis, dryness, discomfort and pain, itching and local inflammation of the vaginal mucosa wherein said composition is administered vaginally.

13. Non-therapeutic use of a composition according to any of claims 1-12 to counteract the phenomena of irritation and dysbiosis of the vaginal mucosa.

14. Kit comprising the composition according to any of claims 1-12, in a single package or in predefined dosage units, a reusable applicator or a disposable applicator containing effective doses of the composition and instructions for use.

Figure 1

Figure 2

VG = Vaginal Gel S03101 Batch: RS52123, SLS = Sodium lauryl sulfate 0.5%, NC = Negative Control

**Figure 3**

**Figure 4**

Europäisches
Patentamt

European
Patent Office

Office européen
des brevets

# EUROPEAN SEARCH REPORT

**Application Number**

EP 25 18 4629

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | CN 108 452 293 A (GUANGXI DAQING BIOLOGICAL SCIENCE & TECH CO LTD) 28 August 2018 (2018-08-28) * the whole document * ----- | 1-14 | INV. A61K9/00 A61K9/06 A61K47/36 A61P31/00 |
| Y | CN 108 158 995 A (GUANGZHOU RAINHOME PHARM & TECH CO LTD) 15 June 2018 (2018-06-15) * the whole document * ----- | 2-11 | |
| Y | CN 110 664 733 A (HUAOU YANCHUANG BIO TECH SHENZHEN CO LTD) 10 January 2020 (2020-01-10) * the whole document * ----- | 2-11 | |
| Y | US 11 116 788 B2 (ALGAMED THERAPEUTICS A M T LTD [IL]) 14 September 2021 (2021-09-14) * column 1, lines 50-64 * * column 2, lines 39-43 * * column 3, lines 52-67 * * column 3, line 64 – column 4, line 2 * * column 4, lines 26-60 * * column 5, lines 12-47 * * column 6, line 10 – column 7, line 28 * ----- | 1-14 | TECHNICAL FIELDS SEARCHED (IPC) A61K A61P |
| Y | US 2006/105963 A1 (YANG SHU-PING [US] ET AL) 18 May 2006 (2006-05-18) * page 1, paragraph 7 * * pages 3-4, paragraph 40 * * page 4, paragraph 41-44 * * page 5, paragraph 48 * * page 6, paragraphs 54, 58 * ----- -/-- | 2-11 | |

The present search report has been drawn up for all claims

| Place of search | Date of completion of the search | Examiner |
|---|---|---|
| The Hague | 17 November 2025 | Raposo, Antonio |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

## EUROPEAN SEARCH REPORT

**Application Number**

EP 25 18 4629

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | Venables Rebecca Hayley ET AL: "8 Vaginal Formulations" In: "A Practical Guide to Medicine Administration", 11 July 2018 (2018-07-11), Taylor & Francis, XP093231974, pages 1-3, * the whole document * | 14 | |

-----

TECHNICAL FIELDS
SEARCHED (IPC)

The present search report has been drawn up for all claims

| Place of search | Date of completion of the search | Examiner |
|---|---|---|
| The Hague | 17 November 2025 | Raposo, Antonio |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
 
& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 25 18 4629

This annex lists the patent family members relating to the patent documents cited in the above-mentioned European search report.
The members are as contained in the European Patent Office EDP file on
The European Patent Office is in no way liable for these particulars which are merely given for the purpose of information.

17-11-2025

| Patent document cited in search report | | Publication date | Patent family member(s) | | Publication date |
|---|---|---|---|---|---|
| CN 108452293 | A | 28-08-2018 | NONE | | |
| CN 108158995 | A | 15-06-2018 | NONE | | |
| CN 110664733 | A | 10-01-2020 | NONE | | |
| US 11116788 | B2 | 14-09-2021 | EP | 3204016 A1 | 16-08-2017 |
| | | | US | 2016220601 A1 | 04-08-2016 |
| | | | US | 2019175640 A1 | 13-06-2019 |
| | | | WO | 2016056001 A1 | 14-04-2016 |
| US 2006105963 | A1 | 18-05-2006 | EP | 1809269 A1 | 25-07-2007 |
| | | | KR | 20070084186 A | 24-08-2007 |
| | | | US | 2006105963 A1 | 18-05-2006 |
| | | | WO | 2006053170 A1 | 18-05-2006 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 108452293 A
- CN 108158995 A